# EUROPEAN PATENT SPECIFICATION

(11) **EP 2 595 598 B1**
(45) Date of publication and mention of the grant of the patent: **06.12.2017**
(21) Application number: 11735642.8
(22) Date of filing: 20.07.2011
(51) Int. Cl.: A61K 8/365, A61Q 5/10, A61Q 7/00

(54) **TOPICAL USE OF STEVIOL OR ISOSTEVIOL IN HAIR CARE**
TOPISCHE VERWENDUNG VON STEVIOL ODER ISOSTEVIOL FÜR DIE HAARPFLEGE
UTILISATION TOPIQUE DU STÉVIOL OU DU ISOSTÉVIOL DANS LES SOINS CAPILLAIRES

(30) Priority: 03.03.2011 EP 11156841; 20.12.2010 EP 10195970; 25.10.2010 EP 10013930; 23.07.2010 EP 10170618
(43) Date of publication of application: 29.05.2013
(73) Proprietor: DSM IP Assets B.V., 6411 TE Heerlen (NL)
(72) Inventor: GORALCZYK, Regina, CH-4002 Basel (CH); GRAEUB, Remo, CH-4002 Basel (CH); MAYNE-MECHAN, Annis Olivia, CH-4002 Basel (CH); PIUSSI, Jenny, CH-4002 Basel (CH); RIEGER, Henry, CH-4002 Basel (CH); MOHAJERI, Hasan, CH-4002 Basel (CH)
(74) Representative: Berg, Katja
(86) International application number: PCT/EP2011/062425
(87) International publication number: WO 2012/010624

(56) References cited:
- EP-A1- 0 711 541
- WO-A1-2009/071277
- WO-A1-2011/009863
- JP-A- 10 036 229
- US-A- 5 110 801
- US-A- 5 250 300
- US-A1- 2005 186 233
- DATABASE WPI Week 200936 Thomson Scientific, London, GB; AN 2009-J60481 XP002621394, -& JP 2009 107941 A (HOSOKAWA FUNTAI GIJUTSU KENKYUSHO KK) 21 May 2009 (2009-05-21)
- DATABASE WPI Week 199338 Thomson Scientific, London, GB; AN 1993-299525 XP002621397, -& JP 5 213721 A (SANSHO PHARM CO LTD) 24 August 1993 (1993-08-24)
- IVANA S MARKOVIC ET AL: "Chemical composition of leaf extracts of Stevia rebaudiana Bertoni grown experimentally in Vojvodina", JOURNAL FO THE SERBIAN CHEMICAL SOCIETY, vol. 73, no. 3, 1 January 2008 (2008-01-01), pages 283-297, XP055377422,
- KATARZYNA MARCINEK ET AL: "STEVIA REBAUDIANA BERTONI - CHEMICAL COMPOSITION AND FUNCTIONAL PROPERTIES", ACTA SCIENTIARUM POLONORUM TECHNOLOGIA ALIMENTARIA, vol. 14, no. 2, 1 January 2015 (2015-01-01), pages 145-152, XP055377432, ISSN: 1644-0730

## Description

The present invention relates to the use of steviol and/or isosteviol, and/ or a salt, for application to skin, skin having hair, scalp, hair of a human, or fur of an animal for the overall enhancement of the hair, particularly for restoring hair color and delaying the onset of greyness in hair, lessening hair loss, restoring hair growth after the onset of baldness has occurred, increasing the thickness of hair, or delaying the onset or severity of age-associated hair loss, maintaining of the natural hair color. This invention also relates to a method for stimulating hair growth, and/or preventing the graying of hair, or restoring or maintaining the natural hair color.

### BACKGROUND OF THE INVENTION

The hair is composed of a protein called keratin. The hair itself is arranged in three layers, an outer cuticle, middle cortex and central medulla. If the hair is colored, it is due to the presence of pigments - either eumelanin (black or brown) or pheomelanin (red or yellow). If these pigments are lacking, the hair is white. Canites is the term given to grey hair, it is an illusion created by the mixture of white and colored hairs. Hair grows from a follicle. The walls of the follicle form the outer root sheath of the hair. The lower part of the follicle widens out to form the hair bulb that contains the germinal matrix, the source of hair growth. Dermal tissue projects into the follicle base to form the dermal papilla, and this has a network of capillary blood vessels to supply oxygen, energy, and the amino-acids needed for growth.

Correcting the effects of aging as far as possible is a preoccupation of ever-increasing importance.

It therefore remains a long awaited need in the hair care industry to prevent hair loss, to stimulate hair growth, to prevent age related graying of the hair, to prevent loss of natural coloration of the hair, and to promote restoration of the natural hair color.

Stevia extracts have been described in (JP 11193219 A) as having hair growing and regeneration effect, however, steviol as well as phloretin, which are known inhibitors of cellular glucose transport have been claimed to also inhibit hair growth when applied topically (EP 0 711 541 A1).

JP-2009107941 and JP-10036229 both disclose the use of stevia extract for hair growth, hair restoring effect and for preventing grey hair.

US-525300 discloses oral compositions of fermented condensate of stems of stevia for livestock industry.

EP-711541, US-5110801, WO2011/009863 and WO2009/07127 all disclose compositions comprising steviol and/ or isosteviol suitable for application to the hair in a carrier.

### DETAILED DESCRIPTION OF THE INVENTION

The inventors of the present application now surprisingly found that steviol and/or isosteviol and derivatives as described below with formula (I) have a great potential for use in topical hair care applications for stimulating hair growth, preventing hair loss, and enhancing the overall appearance, eg. thickness, volume, shininess and glossiness of hair of an animal including human. Thus, one aspect of this invention is a method of enhancing an animal's, including a human's, hair comprising administering a topically applied composition comprising steviol and/or isosteviol, for a time sufficient and in an amount effective to enhance the overall appearance, eg. thickness, volume of hair in an animal including human, and observing or appreciating the result.

Another embodiment of this invention is the use of steviol and/or isosteviol in the manufacture of topical or cosmetic composition which stimulates hair growth and enhances the overall appearance of hair of an animal including human, eg. thickness, volume.

In a further embodiment, the compound of formula (I) below is combined with at least one additional active substance selected from the group consisting of: antioxidants, light screening agents, colorants, and biological actives.

Therefore, the present invention provides the topical use of a compound of formula (I) or a salt thereof according to the claims 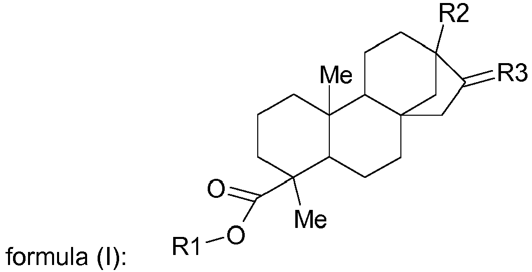 for enhancement of the appearance of hair of an animal including human,
wherein the enhancement of the appearance of hair is selected from the group consisting of: restoring hair color and delaying the onset of greyness in hair, restoring hair growth after the onset of baldness has occurred, or delaying the onset or severity of age-associated hair loss, maintaining of the natural hair color.

It is to be understood that formula (I) as depicted above encompasses all possible stereoisomers.

R1 is hydrogen, R2 is hydroxy, and R3 is CH₂, or R2 is methyl and R3 is O corresponding to the compound of formula (II) steviol (CAS number: 471-80-7) and the compound of formula (III) Isosteviol (CAS number 27975-19-5).

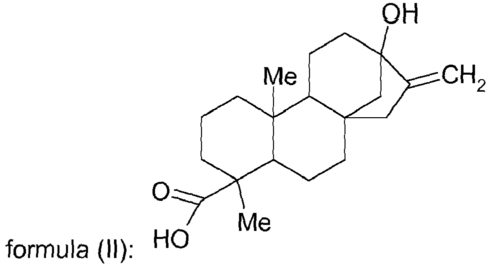

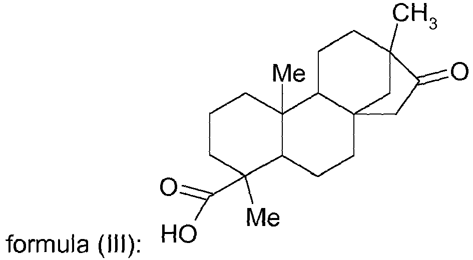

Even more preferred compounds according to the present invention are steviol and isosteviol with the stereochemistry as depicted in formula (IV) and formula (V).

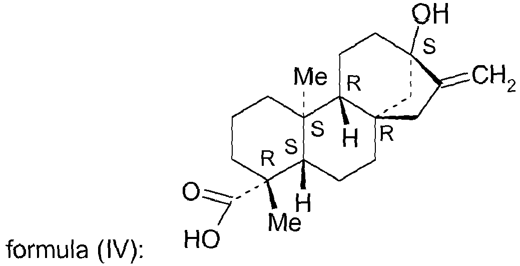

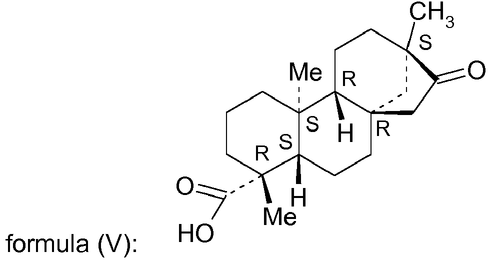

Most preferred compound for all embodiments of the present invention is steviol.

The salts of steviol and/or isosteviol may be formed by any cosmetically acceptable cation which means any metal cation as well as any organic cation that is not toxic to the skin and/or does not cause allergic reactions. Examples of such cations are ammonium salts and alkyl ammonium salts, alkali cations such as sodium and potassium ions and alkaline earth metal cations such as calcium and magnesium ions.

Steviol, isosteviol and salts thereof can then be easily transported to hair follicles.

The compounds used according to the present invention can either be sourced from chemical suppliers like e.g. Sigma or can be prepared by chemical synthesis according to known methods to a person skilled in the art such as e.g.: by degly-cosylation of the respective glucosides (e.g.: stevioside or rebaudiosides A and C) and, in case of the salts, salt formation. Such methods are well known in the art and are e.g.: disclosed in Yingyong Huaxue (1993), 10(4), 35-8: Synthesis of steviol derivatives and their bioactivity, Russian Journal of General Chemistry (2009), 79(10), 2197-2200: O-Alkylation of diterpenoid steviol in the system KOH-DMSO, or Tao et al. Synthesis and bioactivity of Isosteviol derivatives; Chinese Chemical Letters (2005) 16: 1441-1444.

The compositions according to the invention are especially attractive, since many people, including animal owners and handlers, have a special interest in cosmetic treatments considered as "natural" with mild effects and without major side effects.

As used throughout the specification and claims, the following definitions apply:
The term "effective amount" means an amount necessary to obtain a desired physiological effect.

The term "Hair" as used according to the invention refers to both, hair of a human being as well as animal fur.

The term 'hair of an animal including human' relates to all parts of the body of an animal as well as of a human having hair such as the fur of animals as well as the eyelashes, the eyebrows, the beard or the scalp hair of a human. Most preferably the term relates to the hair on the scalp of humans (male or female of any age).

The term "skin having hair" relates to all parts of the skin of a human having hair such as in particular the scalp and the face (eyelashes, the eyebrows, beard). Most preferably the topical compositions are applied to the scalp of humans (male or female of any age).

"Topical composition" as used herein denotes any composition suitable for the topical application to mammalian keratinous tissue such as skin having hair, particularly to the human scalp or to animal skin having fur.

"Preventing" as used herein is not intended to mean that the event will never occur, but means delaying the onset of the condition or event, and/or lessening the severity of the condition or event when it does occur.

"Chronic administration" is meant to convey that administration of the active ingredient regularly occurs over an extended period of time, for example once or twice per day for a time of at least about two weeks, preferably for at least one month, and more preferably at least two months. Alternatively, the regular administration can be every two days, every three days, or once per week or twice per week.

"Extended period of time" means substantially daily for a period of time of at least about two weeks, preferably at least about a month, and even more preferably for at least about two months.

"Observing" or "appreciating" may be done by either the individual who administers or applies the active ingredient topically, or may be done by a third party. The post-administration condition may be compared with the pre-administration condition and analyzed either using a standard test, or by subjective analysis.

"Enhanced Appearance" means that the hair has improved at least one of the following qualities:
∘ Color (i.e. retention/restoration of natural color vs. greyness),
∘ Hair alopecia (i.e. hair is retained, hair loss is stopped or slowed; or hair is regrowing),
∘ Hair has more volume.

In a preferred embodiment, the invention provides the use of compounds of formula (I) according to the present invention for the enhancement of the appearance of hair, wherein the enhancement of the appearance of hair is selected from the group consisting of: lessening hair loss, restoring hair growth after the onset of baldness has occurred, or delaying the onset or severity of age-associated hair loss. More preferably, the enhancement of the appearance of hair is selected from the group consisting of: restoring hair growth after the onset of baldness has occurred.

Compounds of formula (I) according to the present invention may either be used in therapeutic or non-therapeutic topical applications.

In a preferred embodiment, the use according to the present invention is non therapeutic.

In a further embodiment, the compound of formula (I) is combined with at least one additional active substance selected from the group consisting of antioxidants, light screening agents, colorants, and biological actives.

### Antioxidants

Based on the invention all known antioxidants usually formulated into hair care compositions can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinesulfoximine, buthioninsulfone, penta-, hexa-, heptathioninsulfoximine) in very low compatible doses (e.g. pmol to µmol/ kg), additionally (metal)-chelators (such as α-hydroxyfatty acids (citric acid, lactic acid, malic acid), palmic-, phytinic acid, , lactoferrin), β-hydroxyacids, huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, tocopherol and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylideneglucitol, carnosine, butylhydroxytoluene, butylhydroxyanisole, trihydroxybutyrophenone, urea and its derivatives, mannose and derivatives, zinc and derivatives (e.g. ZnO, ZnSO₄), selen and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients, or enzymes such as superoxide dismutase, catalase or similar, or activators of such enzymes. One or more preservatives/ antioxidants may be present in an amount of at least 0.01 wt.-% of the total weight of the composition. Preferably about 0.01 to about 10 wt.-% of the total weight of the composition of the present invention is present. Most preferred, one or more preservatives/ antioxidants are present in an amount about 0.1 to about 1 wt. -%.

### Light screening agents

Light screening agents are advantageously selected from UV-A, UV-B and/or broadband filters. Examples of UV-B or broad spectrum screening agents, i.e. substances having absorption maximums between about 290 and 340 nm may be organic or inorganic compounds. Organic UV-B or broadband screening agents are e.g. acrylates such as 2-ethylhexyl 2-cyano-3,3-diphenylacrylate (octocrylene, PARSOL® 340), ethyl 2-cyano-3,3-diphenylacrylate and the like; camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4,2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0 895 776; organosiloxane compounds containing benzmalonate groups as described in the European Patent Publications EP 0 358 584 B1, EP 0 538 431 B1 and EP 0 709 080 A1 such as polysilicone-15 (PARSOL® SLX); drometrizole trisiloxane (Mexoryl XL); imidazole derivatives such as e.g. 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of prim., sec. and tert. amines like monoethanol amine salts, diethanol amine salts and the like; salicylate derivatives such as isopropylbenzyl salicylate, benzyl salicylate, butyl salicylate, ethylhexyl salicylate (PARSOL® EHS, NEO Heliopan OS), isooctyl salicylate or homomenthyl salicylate (homosalate, PARSOL® HMS, NEO Heliopan OS) and the like; triazine derivatives such as ethylhexyl triazone (Uvinul T-150), diethylhexyl butamido triazone (Uvasorb HEB). Encapsulated UV-filters such as encapsulated ethylhexyl methoxycinnamate (Eusolex UV-pearls) or microcapsules loaded with UV-filters as e.g. disclosed in EP 1 471 995 and the like. Inorganic compounds are pigments such as microparticulated TiO₂, ZnO and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.

Examples of broad spectrum or UV A screening agents i.e. substances having absorption maximums between about 320 and 400 nm may be organic or inorganic compounds e.g. dibenzoylmethane derivatives such as 4-tert. butyl-4'-methoxydibenzoyl-methane (PARSOL® 1789), dimethoxydibenzoylmethane, iso-propyldibenzoylmethane and the like; benzotriazole derivatives such as 2,2'-methylene-bis-(6-(2H-benzotriazole-2-yl)-4-(1,1,3,3,-tetramethylbutyl)-phenol (TINOSORB M) and the like; bis-ethylhexyloxyphenol methoxyphenyl triazine (Tinosorb S) and the like; phenylene-1,4-bis-benzimidazolsulfonic acids or salts such as 2,2-(1,4-phenylene)bis-(1H-benzimidazol-4,6-disulfonic acid) (Neoheliopan AP); amino substituted hydroxybenzophenones such as 2-(4-diethylamino-2-hydroxybenzoyl)-benzoic acid hexylester (Uvinul A plus) as described in EP 1 046 391; Ionic UV-A filters as described in WO 2005/080341 A1; pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art. As dibenzoylmethane derivatives have limited photostability it may be desirable to photo-stabilize these UV-A screening agents. Thus, the term "conventional UV-A screening agent" also refers to dibenzoylmethane derivatives such as e.g. PARSOL® 1789 stabilized by, e.g. 3,3-Diphenylacrylate derivatives as described in EP 0 514 491 B1 and EP 0 780 119 A1; Benzylidene camphor derivatives as described in the US Patent No. 5,605,680; Organosiloxanes containing benzmalonate groups as described in the EP 0 358 584 B1, EP 0 538 431 B1 and EP 0 709 080 A1.

### Colorants

Based on the invention, all colorants usually formulated into hair care compositions which have an absorption in the visible light of electromagnetic radiation (400 nm to 800 nm) can be used. The absorption is often caused by the following chromophores: Azo- (mono-, di-, tris-, or poly-)stilbene-, carotenoide-, diarylmethane-, triarylmethane-, xanthene-, acridine-, quinoline-, methine- (also polymethine-) thiazol-, indamine-, indophenol-, azin-, oxazine-, thiazine-, anthraquinone-, indigo-, phthalocyanin and further synthetic, natural and/or inorganic chromophores.

FD & C and D & C which can be used in hair care compositions according to the invention are e.g. curcumin, riboflavin, lactoflavin, tartrazine, quinoline yellow, cochenille, azorubin, amaranth, ponceau 4R, erythrosine, red 2G, indigotin, chlorophyll, chlorophyllin, caramel, carbo medicinalis, carotenoids, carotin, bixin, norbixin, annatto, orlean, capsanthin, capsorubin, lycopin, xanthophyll, flavoxanthin, lutein, kryptoaxanthin, rubixanthin, violaxanthin, rhodoxanthin, canthaxanthin, betanin, anthocyans without being limited thereto. Examples of dyes are e.g. inorganic pigments such as iron oxide (iron oxide red, iron oxide yellow, iron oxide black etc.) ultramarines, chromium oxide green or carbon black. Other colorants and dyes which can be used in the compositions according to the invention comprise natural or synthetic organic pigments, disperse dyes which may be solubilized in solvents like direct hair dyes of the HC type, for example HC red No. 3, HC Blue No. 2 and all other hair dyes listed in International Cosmetic Ingredient Dictionary Handbook, 11th edition, 2006) or the dispersion dyes listed in Color Index International Society of Dyers and Colorist, color varnishes (insoluble salts of soluble dyes, like many Ca-, Ba- or Al-salts of anionic dyes), soluble anionic or cationic dyes such as acid dyes (anionic), basic dyes (cationic), direct dyes, reactive dyes or solvent dyes, fluorescent dyes, fluorescein and isothiocyanates.

### Biological actives.

Biological actives are advantageously selected from general activators of melanogenesis like tyrosinase activators, peptide hormones, cAMP-activators (caffeine) and neurotrophins.

Preferred tyrosinase activators are any substance which increases tyrosinase expression or enzyme activity, like e.g. glycyrrhizin from the root of licorice.

Peptide hormones belonging to the group of melanocortins are the preferred peptide hormones including ACTH, alpha-MSH, beta-MSH and gamma-MSH; these peptides are all cleavage products of a large precursor peptide called proopiomelanocortin (POMC). Alpha-MSH is the most important melanocortin for pigmentation. The melanocyte-stimulating hormones (collectively referred to as MSH or intermedins) are a class of peptide hormones that in nature are produced by cells in the intermediate lobe of the pituitary gland. They stimulate the production and release of melanin (melanogenesis) by melanocytes in skin and hair. Therefore, they will be advantageously combined with the compounds of the present invention.

In yet another embodiment, the compounds of the present invention are used topically on hair, wherein hair is a mammal's fur. Preferred mammals are horses, dogs and cats.

In a further embodiment, the compound of formula (I) is used in an effective amount selected in the range of 0.00001 to 20 wt.-%, preferably, 0.0001 to 5 wt.-%, more preferably, 0.001 to 0.5 wt.-% based on the total weight of the topical composition.

According to the present invention, the compound of formula (I) is used in a topical composition in an amount in the range of 0.0001 to 5 wt.-%, more preferably, 0.001 to 0.5 wt.-% based on the total weight of the topical composition.

The present invention also relates to the use of compounds of formula (I) for increasing the total number of melanocytes in hair follicles and/or for increasing the differentiation and migration of melanocytes from the hair sheath to the hair matrix and/or increasing proliferation of hair cells, and/or increasing the production of melanin, and/or for lengthening hair growth phase (anagen) and/or shortening hair resting phase (telogen). Number of melanocytes in the hair follicle can be evaluated by standard methods e.g. immunohistochemical staining of the melanocytes with the pan-melanocyte marker NKI-beteb following by counting the NKI-beteb+ cells.

Differentiation and migration of melanocytes from the hair sheath to the hair matrix can be measured by immunohistochemical staining of c-kit+ melanocytes in the hair follicle, and by assessing their distribution within the hair follicle.

In another embodiment, the invention further provides the use of a compound of formula (I) according to the present invention, in combination with the use of other ingredients which are conventionally used in topical compositions, such as in particular hair care compositions, generally enhance the appearance of hair, eg. thickness, volume, shininess and glossiness, to prevent the graying of hair and/or restore or maintain the natural hair color, such as 5,6-dihydroxyindoline HBr, 5,6-dihydroxyindoline HBr in combination with 2-methylresorcinol and/or arginine.

The present invention further provides the use of compounds of formula (I) as described in this invention for elongation of hair follicles, increasing the proliferation of hair follicle cells (outer/inner root sheet, dermal papilla fibroblasts).

Thus, the present invention provides the use of compound of formula (I) for restoring hair color and delaying the onset of greyness in hair, and/or maintaining of the natural hair color.

In another embodiment, this invention relates to a cosmetic non-therapeutic method for stimulating hair growth, and/or preventing the greying of hair or restoring or maintaining the natural hair color comprising the steps of applying to skin having hair, for a sufficient time, a topical composition comprising an effective amount a compound of formula (I), 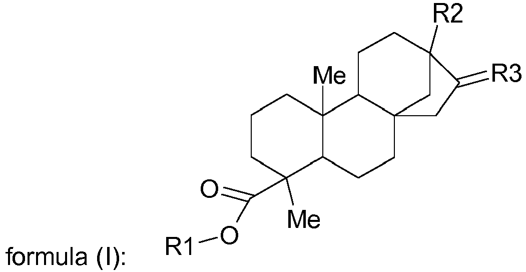 wherein
R1 is hydrogen, R2 is hydroxy, and R3 is CH₂, or R1 is hydrogen, R2 is methyl and R3 is O
   and observing the result. Compounds are steviol and isosteviol in any isomeric form, most preferred are compounds of formula (IV) and formula (V), even most preferred is steviol. Amounts of the compound of formula (I) are selected in the range of 0.0001 to 5 wt.-%, more preferably, 0.001 to 0.5 wt.-% based on the total weight of the topical composition.

Preferably, the topical composition for the method according to the present invention is a hair care composition, and more preferably, it is a hair tonic, a conditioner, a shampoo, or a styling gel. The topical composition may further comprise at least one additional active substance selected from the group consisting of antioxidants, light screening agents, colorants, and biological actives.

### Graying of the Hair

One embodiment of this invention is the prevention of the graying of hair for restoration and/or maintenance of the natural hair color, as shown by the ability of said compounds and derivatives to increase total number of melanocytes in hair follicles, as well as, to increase the differentiation and migration of melanocytes from the hair sheath to the hair matrix.

This invention relates to a method for preventing the graying of hair, delaying the onset of graying, and/or restoring and/or maintaining the natural hair color which comprises the step of applying a topical composition comprising an effective amount of steviol and/or isosteviol, or a salt, an ester, a diester, or an ether thereof as depicted in formula (I) to human or animal skin having hair, and observing the prevention, restoration, or maintenance of natural hair color.

Thus the present invention also encompasses a method of topically administering an amount of steviol and/or isosteviol or a salt, an ester, a diester, or an ether thereof and observing or appreciating an increase in the number of melanocytes in hair follicles, and/or increasing the differentiation or migration of melanocytes from the hair sheath to the hair matrix. The observation or appreciation can be done by noticing a decrease in grayness, maintenance of original hair color, or by observing a restoration of original hair color.

The efficacy of the use of compounds of formula (I) in accordance with the present invention for prevention of the greying of hair and/or for restoration and/or maintenance of the natural hair color can be shown by procedures described below:
As a reference (control) a hair tress containing approximately 100 hairs is cut neatly above the scalp. The color of the hair within the tress is measured from the near-root part to the tip. This could be either done 1) visually by scoring, 2) with high density photo documentation and scoring, 3) by pigment analysis and determination of the melanin content from hair following hair degradation and melanin extraction. In this later case, melanin can be measured by photometric means, or by chemical reaction (i.e. formation of pyrrole-2,3,5 tricarboxylic acid from eumelanin, and formation of aminohydroxyphenylalanine isomers for pheomelanin, followed by quantitative chromatographic, spectroscopic, or spectrophotometric analysis. It can also be done directly by assessing the pigment status of the hair bulb of plucked hair using light microscopy and the Lickert-Scale of pigmentation.

A sample of a topical composition (2 to 10 mL or mg/ cm², depending on the type of formulation; preferably a leave-on product such as hair tonic, lotion or cream) containing a preferred amount of a compound of formula (I) is then applied at least once a day on the scalp, typically from 1 to 4 times daily for at least three months, especially six months (because normal hair growth rate is about 1 cm/ month) and distributed equally with a massage on the scalp. In one method, the product is not washed out after application. At the end of the treatment period, a second hair sample is taken from the same place on the scalp and analyzed as described above.

A comparison of the melanin content, hair color or degree of graying is made intra-individually before and after the treatment period.

### Hair Loss

Also according to this invention, topical applications of steviol and/or isosteviol can:
o lessening hair loss,
∘ restoring hair growth after the onset of baldness has occurred,
∘ preventing or counteracting premature hair loss,
∘ delaying the onset or severity of age-associated hair loss, and/or

Thus another aspect of this invention is a method of enhancing hair appearance selected from the group consisting of:
∘ lessening of hair loss,
∘ restoring hair growth after the onset of baldness has occurred,
∘ preventing or counteracting premature hair loss,
∘ delaying the onset or severity of age-associated hair loss, and/or
comprising topically administering steviol and/or isosteviol, and observing the enhancement.

The present invention also illustrates the use of steviol and/or isosteviol or a salt, thereof as depicted in formula (I) for stimulating dermal papilla region and keratinocytes within the hair follicle in order to increase the total number of keratinocytes responsible for hair growth and/or releasing growth stimulating molecular signals from surrounding cells of the hair follicle and/or migration of progenitor keratinocytes to rebuild hair follicles.

### Veterinary Uses

In another aspect of this invention, the topical formulation of a compound of formula (I) is administered to a non-human animal, which is preferably a mammal.
In a preferred aspect of this invention, the non-human animal is a mammal, such as a companion animal (dog, cat, ferret) or an animal which is used in the fur industry (minks, chinchillas or the like), or an animal which is shown in competition (such as dogs, horses, cats, rabbits and other farm animals). Supplementing the animal's normal bathing or grooming regime with steviol and or isosteviol, a salt, an ester, a diester, or an ether -containing compositions of this invention will enhance the appearance of the animals' fur. Thus another aspect of this invention is a veterinary topical composition containing a fur-enhancing amount of steviol and/or isosteviol or a salt thereof.

Another aspect of this invention is a shampoo or other topical formulation especially designed for a show animal which comprises steviol and/or isosteviol or a salt thereof. This should be applied to the animal daily for at least one month, and preferably for at least two months prior to the competition in order for its fur to be at its optimal condition.

In a further embodiment the invention relates to the use of a compound of formula (I) or a salt thereof for fur grooming (so the fur no longer appears neglected, and looks better groomed).

### Dosages

The physiological effect may be achieved by one single dose or by repeated doses. The dosage administered may, of course, vary depending upon known factors, such as the physiological characteristics of the particular composition; the age, health and weight of the recipient; the nature and extent of the symptoms; the kind of concurrent treatment; the frequency of treatment; and/or the effect desired and can be adjusted by a person skilled in the art. The topical compositions contain the compound of formula (I) according to the present invention in an amount of from 0.0001 to 5 wt.-%, even more preferably in an amount from 0.001 to 0.5 wt.-%, based on the total weight of the composition.

The topical compositions are applied at least several times per week, preferably at least once per day, and more preferably applied at least twice a day such as e.g. once in the morning and once in the evening. Applications should be for a chronic period of time, i.e. at least one week, preferably for at least two weeks, and more preferably for at least 4 weeks in order to observe results.

### Formulations

The present invention exemplifies a hair care composition comprising a compound of formula (I), 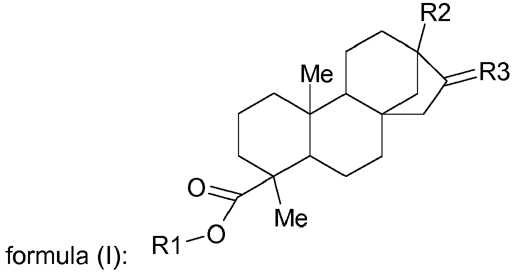 wherein
R1 is hydrogen, R2 is hydroxy, and R3 is CH₂, or R1 is hydrogen, R2 is methyl and R3 is O
and at least one conventional hair care carrier.

In a preferred embodiment, the present invention is also exemplifying a hair care composition comprising a compound of formula (I) as described above, and at least one conventional hair care carrier, wherein the pH of the composition is below 7, preferably below 6, more preferably below 5, even more preferably below 4.

In another embodiment, the hair care composition according to the present invention further comprises an antioxidant selected from the group of antioxidants usually formulated in hair care, and as described above, and/or, a light screening agent selected from UV-A, UV-B, and/or broad band UV filters as described in the present application.

The present invention is also exemplifying a hair care composition comprising a compound of formula (I), 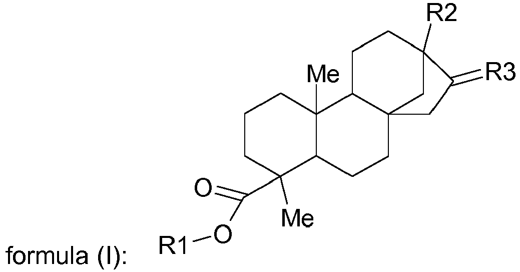 wherein
R1 is hydrogen, R2 is hydroxy, and R3 is CH₂, or R1 is hydrogen, R2 is methyl and R3 is O
and at least one conventional hair care carrier, and an antioxidant selected from the group of antioxidants usually formulated into hair care and/or a light screening agent selected from UV-A, UV-B and/or broadband filters as described in the present application.

The present invention further provides a composition as described above which is a gel, a lotion, a tincture, a spray, a mousse, a cleansing composition, a shampoo, or a foam.

In particular the topical compositions are hair care compositions such as conditioners, treatments, tonics, styling gels, mousses, shampoos, hair sprays, pomades, setting lotions, coloring and permanent waving compositions. Of particular interest for the purpose of the present invention are tonics, conditioners, treatments, and styling gels which may be in the form of a gel, a lotion, a tincture, a spray, a mousse, a cleansing composition or a foam and which may be applied according to individual needs, e.g., once daily as a lotion, tincture, mousse or spray; or once or twice weekly as a conditioner or treatment.
The typical composition used in the method for preventing the graying of hair as well as for restoring and/or maintaining the natural hair color according to the present invention may further comprise other ingredients which are conventionally used in topical compositions such as 5,6-dihydroxyindoline HBr, 5,6-dihydroxyindoline HBr in combination with 2-methylresorcinol and/or arginine.

In accordance with the present invention, a compound of formula (I) with the definitions and preferences as given above is useful in topical compositions such as in particular hair care compositions which further contain carriers and/or excipients or diluents conventionally used in topical, respectively, hair care compositions.

The compound of formula (I) may be combined with suitable auxiliary agents which are conventionally used in hair care compositions such as disclosed in general terms in Ullmann's Encyclopedia of Industrial Chemistry (1989), Vol. A 12, Hair Preparations, and more specifically, e.g., in International Patent Application No. WO 00/06094, WO 00/07550 and WO 01/06994.

Thus, the use of a compound of formula (I) according to the present invention may be combined with the use of further ingredients to protect the hair against detrimental environmental impact and to improve the health of the hair.

The compounds of formula (I) according to the present invention may be incorporated into conventional hair care compositions as described below:
The hair care compositions may comprise additional cosmetic or dermatological adjuvants and/or additives (cosmetic carrier) which are preferably selected from
   1.) Water
   2.) Water soluble organic solvents, preferably C1-C4-Alkanols
   3.) Oils, fatty substances, waxes
   4.) Various esters different to 3) of C6-C30 monocarboxylic acids with mono-, di-, or trivalent alcohols
   5.) Saturated acyclic and cyclic hydrocarbons
   6.) Fatty acids
   7.) Fatty alcohols
   8.) Silicone oils
   9.) Surface active ingredients
and mixtures thereof.

The hair care compositions can contain further adjuvants and additives such as preservatives, antioxidants, silicones, thickeners, softeners, anionic, cationic, nonionic or amphoteric emulsifiers, light screening agents, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, light stabilizers, insect repellents, antibacterial agents, or any other ingredients usually formulated into hair care compositions. The necessary amounts of the adjuvants and additives can, based on the desired product, easily be chosen by a skilled artisan in this field and will be illustrated in the examples, without being limited hereto.

Preferably the hair care compositions are in the form of cosmetic hair-treatment preparations, e.g. hair tonics, conditioners, hair-care preparations, e.g. pretreatment preparations, styling creams, styling gels, pomades, hair rinses, treatment packs, intensive hair treatments e.g. leave-on and rinse-off deep conditioners, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidizing dyes, or natural hair colorants, such as henna or chamomile.

Preferred hair care compositions are leave-on compositions selected from hair tonics, conditioners, treatments, and styling gels.

Based on the application the hair care preparations may be in the form of a (aerosol) spray, (aerosol) foam, gel, gel spray, cream, lotion, liquid or a wax. Hair sprays comprise as well aerosol sprays as pump sprays without propellant. Hair foams comprise as well aerosol foams as pump foams without propellant. Hair sprays and hair foams comprise mainly or exclusively water soluble or water dispersible components. If the components used in hair sprays or hair foams according to the invention are water dispersible, then they may be in the form of micro dispersions with particle sizes of usually 1-350 nm, preferably 1-250 nm. The solid content of such preparations is typically in the range of 0.5 to 20 wt.-% of the total weight of the preparation. Such micro dispersions normally do not need further emulsifiers or tensides for their stabilization.

An exemplary hair gel with the compound of the present invention may comprise:
1. 0.1 to 20 wt.-% preferably 1 to 10 wt.-% of at least one hair polymer;
2. 0 to 10 wt.-% of at least one carrier (solvent), selected from C2-C5 alcohols, preferably ethanol;
3. 0.01 to 5 wt.-%, preferably 0.2 to 3 wt.-% of at least one thickener;
4. 0 to 50 wt.-% of a propellant;
5. 0 to 10 wt.-%, preferably 0.1 to 3 wt.-% of a styling polymer different to 1.), preferably a water soluble non-ionic polymer;
6. 0 to 1 wt.-% of at least one refatter, preferably selected from glycerine and glycerine derivatives;
7. 0 to 30 wt.-% of other customary additives e.g. a silicone component
8. 0.005 to 5 wt.-% of a compound of formula (I) according to the present invention,
9. water ad 100 wt.-%

An exemplary conditioner preparation may comprise:
1. 0.05 to 10 wt.-% of a hair polymer
2. 5 to 95 wt.-% of water
3. 5 to 50 wt.-% of surfactant
4. 0 to 5 wt.-% of an additional conditioning agent
5. 0 to 10 wt.-% other customary additives
6. up to 20 wt.-% of a compound of formula (I)
   all ingredients adding up to 100 wt.-%.

An exemplary styling composition with the compound of the present invention may comprise:
1. 0.1 to 10 wt.-% of at least one hair polymer;
2. 20 to 99 wt.-% water and/or alcohol;
3. 0 to 70 wt.-% of at least one propellant;
4. 0 to 20 wt.-% of customary additives;
5. 0.005 to 5 wt.-% of a compound of formula (I) according to the present invention.

An exemplary styling gel with the compound of the present invention may comprise:
1. 0.1 to 10 wt.-% of a hair polymer;
2. 60 to 99.85 wt.-% of water and/or alcohol;
3. 0.05 to 10 wt.-% of a gel former;
4. 0 to 20 wt.-% of other customary additives.
5. 0.005 to 5 wt.-% of a compound of formula (I) according to the present invention.

An exemplary hair care composition (spray) with the compound of the present invention may comprise:
1. 0.005 to 5 wt.-% of a compound of formula (I) according to the present invention,
2. 30 to 99.5 wt.-%, preferably 40 to 99 wt.-%, of at least one solvent chosen from water, water-miscible solvents and mixtures thereof;
3. 0 to 70 wt.-% of propellant;
4. 0.1 to 10 wt.-% of at least one water-soluble or water-dispersible hair polymer
5. 0 to 0.3 % by weight of at least one water-insoluble silicone;
6. 0 to 0.5 wt.-% of at least one wax, preferably at least one fatty acid amide;
7. customary additives.

Another hair care composition with the compound of the present invention may comprise:
1. 0.05 to 20 wt.-% of at least one hair polymer;
2. 20 to 99.95 wt % of water and/or alcohol;
3. 0 to 79.5 wt.-% of customary additives;
4. 0.005 to 5 wt.-% of a compound of formula (I) according to the present invention.

An exemplary composition for aerosol foams with the compound of the present invention may comprise:
1. 0.1 to 10 wt.-% of at least one hair polymer;
2. 55 to 99.8 wt.-% water and/or alcohol;
3. 5 to 20 wt.-% of a propellant;
4. 0.1 to 5 wt.-% of an emulsifier;
5. 0 to 10 wt.-% of customary additives.
6. 0.005 to 5 wt.-% of a compound of formula (I) according to the present invention.

An exemplary shampoo preparation with the compound of the present invention may comprise:
1. 0.05 to 10 wt.-% of a hair polymer;
2. 25 to 94.95 wt.-% of water;
3. 5 to 50 wt.-% of surfactant;
4. 0 to 5 wt.-% of an additional conditioning agent;
5. 0 to 10 wt.-% other customary additives.
6. 0.005 to 5 wt.-% of a compound of formula (I) according to the present invention,
7. 0 to 5 wt.-% opacifiers and/or pearly gloss-imparting substances

The hair care composition according to the invention can comprise at least a water-soluble or water-dispersible hair polymer. Typical hair polymers for use in the present invention are commercially available polymers for hair care such as hair styling or conditioning polymers such as e.g. copolymers of vinyl acetate and crotonic acid, copolymers of methyl vinyl ether and maleic anhydride, copolymers of acrylic acid or methacrylic acid with other monomers, polyurethanes, N-vinylpyrrolidone and silicone polymers.

The content of the hair polymer is generally from about 0.1 to 10 % by weight, based on the total weight of the composition. Here, it is preferable to use water-soluble or water-dispersible polyurethanes which, if desired, additionally comprise siloxane groups in copolymerized form.

The composition according to the invention can further comprise, at least one water-insoluble silicone, in particular a polydimethylsiloxane, e.g. the Abil® grades from Goldschmidt. The content of the silicone is then generally from about 0.0001 to about 2 % by weight, preferably from about 0.001 to about 1 % by weight, based on the total weight of the composition. Preferred waxes according to the present invention are fatty acid amides, such as, for example, erucamide.

The hair care compositions according to the present invention can, where appropriate, additionally comprise an antifoaming agent, e.g. based on silicone. The amount of antifoaming agent is generally up to 0.001 % by weight, based on the total amount of the composition. The compositions according to the invention have the advantage that, on the one hand, they impart the desired hold to the hair and, on the other hand, the polymers are easy to wash out (redispersible). Generally, a natural appearance and shine is imparted to the hair, even when the hair is by its very nature especially thick and/or dark.

The term alcohol refers to all alcohols usually used in cosmetic compositions such as ethanol, n-propanol, isopropanol.

Other ingredients are cosmetic adjuvants and additives such as propellants, antifoaming agents, surface active ingredients e.g. tensides, emulsifiers, foam former and solubilisators. The used surface active ingredients may be anionic, cationic, amphoteric or neutral. Further ingredients may be preservatives, antioxidants, perfume oils, lipidic refatters, active and/or caring ingredients such as panthenol, collagen, vitamins, protein hydrolysates, alpha- and beta hydroxyl carbonic acids, stabilisators, pH regulators, opacifiers, colorants, dyes, gel formers, salts, moisturizers, complex formers, viscosity regulators or light screening agents without being limited thereto.

In order to obtain certain properties the hair care compositions may additionally comprise conditioning compounds based on silicone such as polyalkylsiloxane, polyarylsiloxane, polyarylalkylsiloxane, silicone resins, polyethersiloxane or dimethicone copolyole (CTFA) and amino functionalized silicone compounds such as amodimethicone (CTFA), GP 4 Silicone fluid® and GP 7100® (Genesee), Q2 8220® (Dow Corning), AFL 40® (Union Carbide) or polymers as disclosed in EP 0 852 488. Other suitable ingredients comprise silicone propfpolymers having a polymeric silicone backbone and non-silicone containing side chains or a non silicone containing polymeric backbone and silicone side chains such as Luviflex® Silk or polymers disclosed in EP 0 852 488.

Typical propellants for hair sprays or aerosol foams may be used. Preferred are mixtures of propane/ butane, pentane, dimethylether, 1,1-difluoroethane (HFC-152a), carbon dioxide, nitrogen or compressed air.

All emulsifiers for aerosol foams or surfactants for shampoo preparations may be conventionally used non-ionic, cationic, anionic or amphoteric emulsifiers/surfactants.

Examples of non-ionic emulsifiers are (INCI-nomenclature) Laureths, e.g. Laureth-4; Ceteths, e.g. Ceteth-1, polyethyleneglycolcetylether; ceteareths, e.g. ceteareth-25, polyglycol fatty acid glycerides, hydroxylated lecithins, lactyl esters of fatty acids, alkylpolyglycosides. Examples of non-ionic surfactants are e.g. reaction products of aliphatic alcohols or alkylphenols with 6 to 20 C-Atoms of a linear or branched alkyl chain with ethyleneoxide and/or propyleneoxide. The amount of alkyleneoxide is about 6 to 60 mol to one mol alcohol. Furthermore alkylaminoxide, mono- or dialkylalkanolamide, fatty esters of polyethylene glycols, alkylpolyglycosides or sorbitan ester are suitable for the incorporation of hair care compositions according to the invention.

Examples of cationic emulsifiers/surfactants are quaternised ammonium compounds e.g. cetyltrimethylammoniumchloride or bromide (INCI: cetrimoniumchloride or bromide), stearyl benzyl dimethyl ammonium chloride, distearyldimethylammonium chloride, stearamidopropyldimethylamine, hydroxyethylcetyldimonium phosphate (INCI: Quaternium-44), Luviquat® Mono LS (INCI: Cocotrimoniummethosulfate), poly(oxy-1,2-ethandiyl), (octadecylnitrilio) tri-2, 1-Ethandiyl) tris-(hydroxy)-phosphate (INCI Quaternium-52). Furthermore, cationic guar derivatives such as guarhydroxypropyltrimoniumchloride (INCI) may be used in conditioner/shampoo preparations.

Anionic emulsifiers/surfactants can be selected from alkylsulfate, alkylethersulfate, alkylsulfonate, alkylarylsulfonate, alkylsuccinate, alkylsulfosuccinate, N-alkylsarkosinate, acyltaurate, acylisethionate, alkylphosphate, alkyletherphosphate, alkylethercarboxylate, alpha-olefinsulfonate, especially the alkali-und earth alkali salts, e.g. sodium, potassium, magnesium, calcium, as well as ammonium- and triethanol amine-salts. The alkylethersulfate, alkyletherphosphate and alkylethercarboxylate may comprise between 1 to 10 ethyleneoxide or propyleneoxide units, preferably 1 to 3 ethyleneoxide-units per molecule.

Suitable anionic surfactants are e.g. sodium laurysulfate, ammonium laury sulfate, sodium laurylethersulfate, ammonium laurylethersulfate, sodium lauroylsarkonisate, sodiumoleylsuccinate, ammonium laurylsulfosuccinate, sodium dodecylbenzolsulfonate, triethanolamidodecylbenzolsulfonate.

Suitable amphoteric surfactants are e.g. alkylbetaine, alkylamidopropylbetaine, alkylsulfobetaine, alkylglycinate, alkylcarboxyglycinate, alkylamphoacetate or propionate, alkylamphodiacetate or dipropionate such as cocodimethylsulfopropylbetaine, laurylbetaine, cocamidopropylbetaine or sodium cocamphopropionate.

As gel formers, all typical cosmetic gel formers can be used such as slightly cross linked polyacrylic acid e.g. Carbomer (INCI), cellulose derivatives, polysaccarides e.g. xanthan gum, caprylic/ capric triglyceride (INCI), sodiumacrylate-copolymers, polyquaternium-32 (and) paraffinum liquidum (INCI), sodiumacrylate-copolymers (and) paraffinum liquidum (INCI) (and) PPG-1 trideceth-6, polyquaternium-37 (and) propyleneglycoldicapratdicarylate (and) PPG-1 trideceth-6, polyquaternium-7, polyquaternium-44.

In order to provide the formulation a pearlescent appearance or to give the impression of a richer or creamier product, the hair care composition may additionally comprise opacifiers and/or pearly gloss-imparting substances, such as soaps or salts of carboxylic acids, cationics including cationic polymers, dimethicone (INCI) or amodimethicone (INCI).

Other customary additives are for example long chain fatty alcohols such as cetyl alcohol, stearyl alcohol, cetylstearyl alcohol, dimethylstearamine. Furthermore the hair care composition may contain lipids such as dimethicone, amodimethicone, mineral oil, or silicon derivatives such as Dimethicone Copolyol.

In another embodiment, the present invention also relates to a dual-vial packaging system as described in WO2008049443 containing the compound of formula (I) as a dried powder in a compartment, and a cosmetically acceptable carrier in the other compartment.

The invention is further illustrated by the Examples which follow without being limited thereto.

### EXAMPLES

### Example 1: Growth of Human Hair Follicle

Human hair follicles were obtained from human skin fragments (obtained by plastic surgery) and grown in supplemented William's E medium supplemented with penicillin/streptomycin, L-glutamine (2 mM), insulin (10 µg/ml and hydrocortisone (8 nM). Hair follicle growth was assessed via length measurements (day 0, 4, 7). Triiodothyronine (T3, 30 µM) was used as positive control. The effect of steviol (at 0.625 to 5 µg/ml) was tested in parallel. At day 7, RNA was extracted from hair follicles that had been subjected to different treatments (control, T3, steviol). RNA was processed for Affymetrix® DNA microarray analysis in order to identify effects of compounds on gene expression. Where appropriate, RNA expression levels were further quantified by RT-PCR using the ABI 7900 Taqman instrumentation. In all procedures the protocols suggested by the manufacturers were strictly followed.

### Results

### Effects on hair follicle growth:

Steviol dose-dependently favoured the elongation of hair follicles. At low concentrations the effect was significant, whereas at higher concentrations the compound did not significantly contribute to growth.

**Table 1**

| **Treatment** | **Concentration** | **Growth (% of control)** | ***p*-value** |
|---|---|---|---|
| Control | - | 100 | - |
| Triiodothyronine | 30 µM | 133 | < 0.01 |
| Steviol | 0.625 µg/mL | 122 | < 0.1 |
| | 1.25 µg/mL | 121 | < 0.1 |
| | 2.5 µg/mL | 113 | Not significant |
| | 5 µg/mL | 117 | Not significant |

### Effect on gene expression:

Global effects of steviol on gene expression were determined by transcriptomics *i.e.* DNA microarray analysis. The obtained data were processed with Genedata software tools. This resulted in a list of genes that had significantly different expression levels in steviol-treated hair follicles (compared to untreated hair follicles). Examples of genes that were differentially up-regulated by steviol and which are involved in epidermal physiology are given in Table 2. Only genes directly or indirectly involved in epidermal proliferation or differentiation and which were at least 2-fold up-regulated are listed.

**Table 2**

| **Gene** | **Ratio of steviol-treated/untreated** | **p - value** |
|---|---|---|
| Transmembrane protein with EGF-like and two follistatin-like domains 2 | 3.10 | 0.0001 |
| Fibroblast growth factor 7 (keratinocyte growth factor) | 2.91 | 0.0006 |
| CASP8 and FADD-like apoptosis regulator | 2.58 | 0.0004 |
| Topoisomerase (DNA) II alpha 170kDa | 2.45 | 0.0004 |
| TIMP metallopeptidase inhibitor 3 | 2.32 | 0.0001 |
| Chemokine (C-X-C motif) ligand 12 (stromal cell-derived factor 1) | 2.22 | 0.0005 |
| Integrin, alpha 6 | 2.15 | 0.0004 |
| Collagen, type IV, alpha 6 | 2.04 | 0.0008 |
| Insulin-like growth factor 1 receptor | 2.00 | 0.001 |

The genes regulated by steviol were further mapped to biological pathways using statistical tools provided with the Genedata software package. Steviol modulated gene expression of the pathways of the epidermal development, ectoderm development and keratinocyte differentiation with a high statistical significance (indicated by p- value in Table 2); other pathways were not significantly affected. This data is in line with the observed effect of steviol on hair follicle elongation (see Table 1) and corroborate that steviol modulates molecular pathways that lead to changes in hair growth.

The mRNA level of some marker genes involved in growth and/or differentiation of cells in the skin and in hair follicle was further determined by RT-PCR and is shown in Table 3.

**Table 3**

| **Gene** | **Treatment of human hair follicle (concentration)** | **Fold changes (versus control)** |
|---|---|---|
| All genes | Control (untreated) | 1 |
| Keratin 6 | Steviol (10 µg/mL) | 1.33 ± 0.11 |
| COX-2 | Steviol (1.25 µg/mL) | 0.46 ± 0.17 |
| Integrin alpha-6 | Steviol (0.625 µg/mL) | 1.62 ± 0.06 |
| HO-1 | Steviol (1.25 µg/mL) | 0.64 ± 0.08 |
| Involucrin | Steviol (1.25 µg/mL) | 0.51 ± 0.22 |

The importance of these genes in epidermal and therefore also hair development is described and discussed in detail *e.g.* by Adriani et al. J. Invest Dermatol 120: 923-931 (2003); Grochot-Preczek et al. Thromb Haemost 104 (on-line 10-June 2010); Li et al. Exp Dermat 9: 431-438 (2000); Ma et al. Ann Acad Med Singapore 33: 784-8 (2004); Neufang et al. Proc Nat Acad Sci (USA) 98: 7629-7634 (2001).

### Example 2: Anti Dandruff Shampoo

| **INCI NOMENCLATURE** | **wt.-%** |
|---|---|
| Aqua | Ad 100 |
| Ammonium Laureth Sulphate (28 %) | 35.00 |
| Ammonium Lauryl Sulphate (25 %) | 15.00 |
| Glycol Distearate | 1.00 |
| Dimethicone | 1.00 |
| Cetyl Alcohol | 0.50 |
| Cocamide MEA | 3.00 |
| Zinc Pyrithione | 1.00 |
| Guar Hydroxipropyltrimonium Chloride | 0.20 |
| Hydrogenated Polydecene | 1.00 |
| Polyquaternium-10 | 0.10 |
| PEG 7m | 0.50 |
| Trimethylpropane Tricaprylate/Tricaprate | 1.00 |
| Preservative | q.s. |
| Fragrance | 0.30 |
| E 104,E 110,E 132 | 0.02 |

| **INCl NOMENCLATURE** | **wt.-%** |
|---|---|
| Compound of formula (I), in particular steviol | 0.01 |

Combine all ingredients and mix intensively until a homogeneous solution is obtained. At the end, add the water under slow agitation and wait until the foam has disappeared.

### Example 3: Conditioning Shampoo

| **INCl NOMENCLATURE** | **wt.-%** |
|---|---|
| Aqua | Ad 100 |
| Sodium Laureth Sulphate (28 %) | 25.00 |
| Cocamidopropyl Betaine (40 %) | 5.00 |
| Sodium Chloride | 2.50 |
| Glycol Distearate | 1.00 |
| Glycerin | 2.00 |
| Dimethiconol | 0.50 |
| Parfum | 0.50 |
| Coco-Glucoside (40%) | 3.00 |
| Carbomer | 0.10 |
| Arginine | 0.05 |
| Glyceryl Oleate | 0.05 |
| Glyceryl Stearate | 1.00 |
| Guar Hydroxypropyltrimonium Chloride | 0.10 |
| Panthenol | 1.00 |
| Disodium EDTA | 0.05 |
| Preservative | q.s. |
| Hydrolyzed Keratin | 0.10 |
| Citric Acid/ Sodium Hydroxide | q.s |
| Compound of formula (I) according to the present invention such as in particular steviol | 0.005 |
| E 102, E 110, FD&C blue | 0.01 |

Combine all ingredients and mix intensively until a homogeneous solution is obtained. At the end, add the water under slow agitation and wait until the foam has disappeared. Then carefully add the thickening agent (sodium chloride).

### Example 4: Shine Shampoo

| **INCl NOMENCLATURE** | **wt.-%** |
|---|---|
| Aqua | Ad 100 |
| Sodium Laureth Sulfate (28%) | 15.00 |
| Disodium Cocoamphodiacetate (40%) | 15.00 |
| Sodium Chloride | 2.00 |
| Glycol Distearate | 1.00 |
| Cocamidopropyl Betaine (40%) | 2.00 |
| Laurdimonium Hydroxypropyl Hydrolyzed Wheat Protein | 1.00 |
| PEG-12 Dimethicone | 1.00 |
| Guar Hydroxypropyltrimonium Chloride | 0.05 |
| Hydrolyzed Wheat Protein | 0.20 |
| Laureth-4 | 1.00 |
| PEG-7 Glyceryl Cocoate | 2.00 |
| Hydrogenated Castor Oil | 1.00 |
| Laureth-2 | 0.50 |
| PEG-55 Propylene Glycol Oleate, | 2.00 |
| Propylene Glycol | 2.00 |
| Mica | 0.20 |
| Citric Acid | 0.01 |
| Parfum | 1.00 |
| E 110, E 104, E 122 | 0.05 |
| Compound of formula (I) according to the present invention such as in particular steviol | 0.05 |

Combine all ingredients and mix intensively until a homogeneous solution is obtained. At the end, add the water under slow agitation and wait until the foam has disappeared. Than add carefully the thickening agent (sodium chloride).

### Example 5: Extra Shine Revitalizing Hair Cream

| | **INCl Nomenclature** | **wt.-%** |
|---|---|---|
| A | Simmondsia Chinensis (Jojoba) Seed Oil | 3.00 |
| | Prunus Armeniaca (Apricot) Kernel Oil | 3.00 |
| | Phenyl Trimethicone | 2.00 |
| | C12-15 Alkyl Benzoate | 2.00 |
| | Glyceryl Stearate SE | 2.00 |
| | Polysilicone-15 | 0.50 |
| | Tocopheryl Acetate | 0.50 |
| | Cetearyl Alcohol | 1.60 |
| B | Aqua | Ad 100 |
| | Compound of formula (I) according to the present invention such as in particular steviol | 0.005 |
| C | Behentrimonium Chloride | 1.00 |
| | Cocodimonium Hydroxypropyl Hydrolyzed Wheat Protein | 0.30 |
| | Propylene Glycol (and) Diazolidinyl Urea (and) Methylparaben (and) Propylparaben | 1.00 |

Heat part A and part B separately to 65 °C under moderate agitation. When both have the same temperature, add part B into part A under agitation. Let cool to 40 °C and add part C under agitation, homogenize. Cool to ambient temperature.

### Example 6: Hair Repair Treatment

| | **INCl NOMENCLATURE** | **wt.-%** |
|---|---|---|
| A | Cetearyl Octanoate | 0.20 |
| | Phytantriol | 0.10 |
| | PEG-40 Hydrogenated Castor Oil | 2.00 |
| B | Parfum | q.s. |
| | Cocotrimonium Methosulfate | 2.00 |
| C | Aqua | Ad 100 |
| D | Polyquaternium-16 | 2.00 |
| | Dimethicone Copolyol | 1.00 |
| | Compound of formula (I) according to the present invention such as in particular steviol | 0.5 |
| | Parfum | q.s. |
| | Alcohol denat. | 10.00 |
| | Citric Acid | q.s. |

Heat Part A to 70 °C. Add part B to part A under stirring. Add the mixture to part C and homogenize. Add part D and let cool down under moderate agitation.

### Example 7: Color Balm

| | **INCl NOMENCLATURE** | **wt.-%** |
|---|---|---|
| A | Ceteareth-6, Stearyl Alcohol | 1.50 |
| | Ceteareth-25 | 1.50 |
| | Cetearyl Alcohol | 3.00 |
| | Cetearyl Octanoate | 6.00 |
| | Phytantriol | 0.30 |
| B | Polyquaternium-44 (13% in water) | 7.70 |
| | Compound of formula (I) according to the present invention such as in particular steviol | 0.005 |
| | Propylene Glycol | 2.00 |
| | Panthenol | 1.00 |
| | Parfum | q.s. |
| | Aqua | Ad 100 |
| C | C.I. 42510, Basic Violet 14 | 0.05 |
| | C.I. 12245, Basic Red 76 | 0.08 |
| | Preservative | q.s. |
| | Citric Acid | q.s. |

Heat parts A and B separately to 70°C. Add part A to B and homogenize. Add part C under stirring.

### Example 8: Silky Hair Cocktail

| | **INCl NOMENCLATURE** | **wt.-%** |
|---|---|---|
| A | Caprylic/Capric Triglyceride (and) Acrylates Copolymer | 3.00 |
| | Dimethicone Copolyol | 0.50 |
| | Dimethicone Copolyol | 2.00 |
| | Cyclomethicone (and) Dimethiconol | 3.00 |
| | Amodimethicone (and) Cetrimonium Chloride (and) Trideceth-10 | 2.00 |
| | Phenyl Trimethicone | 2.00 |
| | Macadamia (Ternifloria) Nut Oil | 1.00 |
| | Tocopheryl Acetate | 0.50 |
| | PEG-40 Hydrogenated Castor Oil | 1.00 |
| | Parfum | q.s. |
| B | Aqua | Ad 100 |
| | Aminomethyl Propanol | 0.46 |
| | Compound of formula (I) according to the present invention such as in particular steviol | 0.01 |
| | PEG/PPG-25/25 Dimethicone/ Acrylates Copolymer (50%) | 4.00 |
| | Preservative | q.s. |

Heat parts A and B separately to 70°C. Add part A to B and homogenize. Let cool down under stirring.

### Example 9: Oil Sheen Moisturizer

| | **INCl NOMENCLATURE** | **wt.-%** |
|---|---|---|
| A | Cetyl Alcohol | 2.00 |
| | PEG-75 Lanolin | 1.00 |
| | Glyceryl Stearate | 4.00 |
| | Ceteareth-25 | 1.00 |
| | Cetearyl Octanoate | 4 |
| B | Glycerin | 10.00 |
| | Compound of formula (I) according to the present invention such as in particular steviol | 0.05 |
| | Propylene Glycol | 2.00 |
| | Cocotrimonium Methosulfate | 1.00 |
| | Trimethylsilylamodimethicone, SM 2115 Octoxynol-40, Isolaureth-6, Glycerin | 1.50 |
| | Polysorbate 20 | 1.00 |
| | Aqua | Ad 100 |
| C | Panthenol | 0.50 |
| | Preservative | q.s. |
| | Parfum | q.s. |
| | Citric Acid | q.s. |

Heat parts A and B separately to 70°C. Add part A to B and homogenize. Add part C under stirring.

### Example 10: Setting Cream High Gloss

| | **INCl NOMENCLATURE** | **wt.-%** |
|---|---|---|
| A | Cetyl Alcohol | 5.00 |
| | Glyceryl Stearate SE | 10.00 |
| | Isopropyl Myristate | 5.00 |
| | Preservative | q.s. |
| | Dimethicone | 1.00 |
| B | Glycerin | 5.00 |
| | Compound of formula (I) according to the present invention such as in particular steviol | 0.05 |
| | Disodium EDTA | 0.20 |
| | PVP | 2.00 |
| | Aqua | Ad 100 |
| C | Parfum | q.s. |

Heat parts A and B separately to 70°C. Add part A to B and homogenize. Add part C under stirring.

### Example 11: Hair Gel

| **INCl NOMENCLATURE** | **wt.-%** |
|---|---|
| Carbomer | 0.50 |
| Aqua | Ad 100 |
| AMP-95 | 0.52 |
| Compound of formula (I) according to the present invention such as in particular steviol | 0.01 |
| PVP | 5.00 |
| Parfum | q.s. |
| PEG-40 Hydrogenated Castor Oil | q.s. |
| Phenoxyethanol (and) Methylparaben (and) Butylparaben (and) Ethylparaben (and) Propylparaben | 0.10 |
| Tocopheryl Actetate | 0.10 |

Disperse carbomer in about 50% of the water amount and add AMP under stirring till a clear gel is formed. Dissolve PVP in the rest of the water. Premix the perfume with PEG-40 hydrogenated castor oil. Mix all parts, and finally add preservative and tocopheryl acetate.

### Example 12: Hair Gel

| **INCl NOMENCLATURE** | **wt.-%** |
|---|---|
| Compound of formula (I) according to the present invention such as in particular steviol | 0.1 |
| Polyquaternium-46 (20%) | 2.50 |
| Alcohol denat. | 15.00 |
| Aqua | Ad 100 |
| Parfum | 0.10 |
| Glycerin | 0.10 |
| Hydroxyethylcellulose | 2.00 |

Combine all ingredients of part 1 and mix intensively until a homogeneous gel is obtained.

### Example 13: Permanent hair tinting formulation

### Part I

| | **INCl Nomenclature** | **wt.-%** |
|---|---|---|
| A | Cetearyl Alcohol | 9.00 |
| | Sodium Ceteaeyl Sulfate | 3.00 |
| | Glyceryl Stearate | 2.50 |
| | Laureth-2 | 2.00 |
| | Stearamide MEA-Stearate | 0.75 |
| | PEG-5 Cocamide | 0.50 |
| | Oleic Acid | 0.50 |
| | Hair Dye | 0.30 |
| B | Aqua | Ad 100 |
| | Ammonium Sulfate | 2.00 |
| | Sodium Sulfite | 0.50 |
| | Disodium EDTA | 0.05 |
| | Compound of formula (I) according to the present invention such as in particular steviol | 0.1 |
| | Ascorbic Acid | 0.50 |
| | Ammonium Hydroxide | 2.50 |

### Part II

| | **INCl Nomenclature** | **wt.-%** |
|---|---|---|
| A | Cetearyl Alcohol | 6.00 |
| B | Aqua | Ad 100 |
| | Hydrogen Peroxide (30%) | 9.00 |
| | Sodium Lauryl Sulfate (28%) | 3.00 |
| | Disodium Phosphate | 0.15 |
| | Phosphoric Acid (85%) | pH 2.0 |

Heat phase A and B of Part I separately to 70 °C. Add phase A to phase B under stirring. Adjust the pH to 11.2.

Heat phase A and B of Part II separately to 70 °C. Add phase A to phase B under stirring. Adjust the pH. Combine Parts I and II shortly before use.

### Example 14: Pharmaceutical Shampoo

### Part I

| | **INCI Nomenclature** | **wt.-%** |
|---|---|---|
| A | Aqua | 50.00 |
| | Compound of formula (I) according to the present invention such as in particular steviol | 5.00 |
| | Methylcellulose | 0.30 |

### Part II

| | **INCI Nomenclature** | **wt.-%** |
|---|---|---|
| A | Sodium Laureth Sulfate (28%) | 44.50 |
| | Ethylparaben | 0.20 |

Dissolve compound of formula (I) according to the present invention (in particular steviol) in water, add Methylcellulose and stir until dissolved; mix Ethylparaben with Sodium Laureth Sulfate.

Mix part 1 with part 2.

### Example 15: Induction of melanin synthesis by Steviol:

### Quantification of melanin synthesis in cell culture:

Normal human melanocytes NHM (HEMn-MP, Clonetics) were seeded in 96 well cell culture plates and grown to sub-confluence for two days in a mixture of M2-Medium (Clonetics) and Medium (Promocell). Culture medium was exchanged with Culture Medium containing Steviol and melanogenesis progressed for another three days at 37 °C with another medium exchange on day two. Including cell layer and culture supernatant the total melanin was extracted using 1.7M KOH with vigorous shaking at RT. We measured melanin content at 405 nm in an absorbance plate reader. The data is normalized to 100 % for the un-treated control sample, and expressed as percentage of the control.

The results are summarized in the table below:

| Control | Glycyrrhizin 1.5 mM | Steviol 0.25 microM | Steviol 0.5 micro M | Steviol 1 micro M |
|---|---|---|---|---|
| 100 % | 132 % | 109 % | 124 % | 130 % |

Glycyrrhizin is used as a positive control since it is a known inducer of melanogenesis. Steviol shows a clear dose dependent positive effect on the production of melanin in human melanocytes.

### Example 16: Silk & Shine Conditioning Fluid:

| **Phase** | **INCI Name** | **% w / w** |
|---|---|---|
| A | Aqua | 51.45 |
| | Stearamidopropyl Dimethylamine | 2.40 |
| | Polyquaternium-67 | 0.15 |
| B | Cetearyl Alcohol | 2.40 |
| | Behenyl Alcohol | 1.80 |
| C | Water, Dimethicone, Polyquaternium- 74, Laureth-7, Sodium Benzoate, Phenoxyethanol | 2.80 |
| | Cyclopentasiloxane & Dimethiconol | 1.00 |
| | Argania Spinosa Kernel Oil | 0.50 |
| D | Aqua | Ad 100.00 |
| | Glutamic Acid | 0.15 |
| | Steviol | 0.50 |
| | Panthenyl Ethyl Ether | 0.75 |
| | Hydrolyzed Sericin | 0.20 |
| | Citric acid | q.s. |
| E | Parfum | 0.60 |
| | Phenoxyethanol & Ethylhexylglycerin | 0.30 |

### Procedure:

- A/B: Heat part A and part B separately to 70°C. When both have reached the same temperature, add part B to part A.
- C: Add. Ingredients of part C and homogenize balanced.
- D: Water phase II cold: Add. all ingredients of part D in water. Add. the cold solution under stirring into the product.
- E: Add. Preservative and Parfum. Homogenize thoroughly and then with continued mixing cool down to ambient temperature.

### Technical Data:

| | |
|---|---|
| pH: 4.8 | Viscosity: 30.000 mPas (# 6/5 RPM) |

### Example 17: Sulfate Free Shampoo:

| **Phase** | **INCI Name** | **% w / w** |
|---|---|---|
| A | Aqua | 50.7299 |
| | Acrylates/Beheneth-25 Methacrylate Copolymer (30 %) | 6.00 |
| B | Sodium Cocoyl Apple Amino Acids (40 %) | 4.00 |
| | Caprylyl/Capryl Glucoside (63 %) | 3.00 |
| | Sodium Lauroyl Glutamate (40 %) | 5.00 |
| | Sodium Cocoamphoacetate (32 %) | 16.00 |
| C | Polyquaternium-22 (40 %) | 1.50 |
| | Panthenol | 0.67 |
| | Calcium Pantothenate | 0.20 |
| | Silica & Titanium Dioxide (Cl 77891) & Tin Oxide (Cl 77861) | 0.05 |
| D | Parfum | 0.80 |
| | Argania Spinosa Kernel Oil | 0.10 |
| | PEG-90M | 0.15 |
| | PEG-18 Glyceryl Oleate/Cocoate | 3.00 |
| | PEG-40 Hydrogenated Castor Oil | 1.40 |
| E | Phenoxyethanol & Ethylhexylglycerin | 0.80 |
| | Coco-Betaine (30 %) | 6.00 |
| | Citric Acid | 0.60 |

### Procedure:

1 Mix part A under stirring.
2 Add ingredients of part B step by step to A under stirring until equal distribution is optioned.
3 Add ingredients of part C step by step to the first part under stirring.
6 Mixing separate: all ingredients of part D and add to the shampoo.
7 Addition ingredients of part E. The batch will thicken.

### Technical Data:

| | | |
|---|---|---|
| pH: 5.9 | Viscosity: 3500-5000 mPas | (Brookfield, #4 / 10 rpm) |

### Example 18: Color Protection Shampoo:

| **Phase** | **INCI Name** | **% w / w** |
|---|---|---|
| A | Aqua | Ad. 100 |
| | Acrylates Copolymer (30 %) | 4.50 |
| B | Sodium Laureth Sulfate (28 %) | 40.00 |
| | Sodium Lauroyl Glutamate (40 %) | 4.00 |
| | Sodium Cocoamphoacetate (32 %) | 5.00 |
| | Sodium Hydroxide (30 %) | 0.20 |
| C | Polyquaternium-7 | 2.00 |
| | Silica & Titanium Dioxide (EU:Cl 77891) & Tin Oxide (EU:Cl 77861) | 0.10 |
| | Peg-4 Distearyl Ether & Sodium Laureth Sulfate & Distearyl Ether & Dicaprylyl Ether | 6.00 |
| | Dimethicone | 0.80 |
| | Saccharide Isomerate | 0.50 |
| | Steviol | 0.25 |
| D | Polysilicone-15 | 0.10 |
| | Phytantriol | 0.20 |
| | Octocrylene & Homosalate & Butyl Methoxydibenzoylmethane & Tocopherol & Glycine Soja (Soyabean) Oil | 0.20 |
| | Parfum | 1.00 |
| E | Coco-Betaine (30 %) | 5.50 |
| F | preservative | 0.80 |
| | Sodium Chloride | 1.00 |

### Procedure:

- A: Mix part A under stirring.
- B: Add. ingredients of part B step by step to part A, under stirring. Neutralize with NaOH.
- C: Add. all ingredients of part C step by step to the mixture.
- D: Mixing separate: ingredients of part D all in perfume and add. into the shampoo.
- E/F: Then add. the thickening agent until desired viscosity is reached. Add preservative.

| | |
|---|---|
| pH: 6.10 | Viscosity: 8.000 mPas (S 4/ 10 Rpm) |

### Example 19: 2-Phase leave-on Conditioner:

| **Phase** | **INCI Name** | **% w / w** |
|---|---|---|
| A | Aqua | 65.50 |
| | Polyquaternium-6 (40 %) | 1.45 |
| | Lactic Acid (10 %) | 0.10 |
| B | Chitosan Glycolate | 5.00 |
| | PEG/PPG-18/18 Dimethicone | 2.00 |
| | Sericin | 1.00 |
| C | Niacinamide | 0.50 |
| | Panthenyl Ethyl Ether | 0.75 |
| | Sodium Benzoate | 0.50 |
| | Steviol | q.n. |
| D | Alcohol | 6.00 |
| | Polysilicone-15 | 1.50 |
| | Octocrylene & Homosalate & Butyl Methoxydibenzoylmethane & Tocopherol & Glycine Soja (Soyabean) | 0.20 |
| | Cyclopentasiloxane | 8.00 |
| | Parfum | 0.50 |
| | Trisiloxane & Dimethicone | 7.00 |

### Procedure:

- A: Add the polymer in water and mix thoroughly until a homogeneous solution is obtained.
Add lactic acid
- B: Add all ingredients of part B successively and mix until clear and uniform.
- C: Add all ingredients of part C successively and mix with moderate propeller agitation.
- D: Mixing separate : all ingredients of part D dissolve in ethanol.
Add the solution into the product.
Homogenize moderate!

### Technical Data:

| | |
|---|---|
| pH: 4.52 | Viscosity: spray able 2-phases |

### Example 20: Solid product which needs to be dissolved prior to the application:

Product forms to treat the hair or the scalp consisting of
a) a solid part
b) a liquid part,
wherein the solid part and the liquid part are mixed prior to application.

The solid part contains Steviol, and the liquid part contains 0 to 100 % water or/and ethanol (0 to 100 %), and/or other compounds required to dissolve the solid part in a comfortable/convenient time (1-8 min), e.g. emulsifiers, glycerine, propylene glycol, butylene glycol, rheological modifier, e.g. Carbomers, Hydroxyethyl cellulose, pH-regulators.

### Composition:

### Solid parts:

| **Nr** | **ingredient** | **Wt-%** |
|---|---|---|
| 1 | Mannitol | 30 |
| 2 | Ascorbyl Glucoside | 20 |
| 3 | Sodium Citrate | 10 |
| | Ceratonia Siliqua Gum | 25 |
| 5 | Steviol | 15 |

### Liquid phase:

| **Nr** | **ingredient** | **Wt-%** |
|---|---|---|
| 1 | Ethanol | 45 |
| 2 | Water | 50 |
| 3 | Sorbitol | 3 |
| | Polysorbate-40 | 0.4 |
| 5 | frangrance | q.n. |

Solid part (0.01 to 10g) are mixed with the liquid part (5 mL-30mL) prior to application in an appropriated system by shaking.

### Example 21: Stabilization of steviol in acidic aqueous solution:

Steviol was dissolved at 1 wt.-% in water and the pH adjusted at different values as shown below. At pH 9, the solution turns brown within 1 day demonstrating instability of Steviol. However, the more acidic the pH, the less discoloration was observed as reported by the patone color index.

Color index of 1 % Steviol solution in water at different pH:

| | **pH 9** | **pH 7** | **pH 6.5** | **pH 6** |
|---|---|---|---|---|
| **Pantone index** | **160** | **158** | **155** | **155** |

### Example 22: Effect on Steviol on hair follicle melanogenesis:

### Material & Method:

For optimal quality of hair follicles with an intact pigmentary-unit, normal human scalp skin hair follicles in the anagen VI stage of the hair cycle were isolated from scalp skin biopsies obtained from elective plastic surgery after obtaining informed consent from patients following the protocol published by Philpott and colleagues with slight modifications (Philpott et al. 1990, Philpott 1999). Briefly, after separation of epidermis and dermis from subcutaneous fat the dermis just above the dermis/subcutis border under a binocular dissecting microscope, the proximal two thirds of anagen hair follicles located in the subcutaneous fat were isolated using watchmakers forceps and subsequently collected in Petri dishes containing complete hair follicle culture medium (Williams E, Biochrom KG seromed, Berlin, Germany); 1 % Penicillin-Streptomycin; 1 % L-Glutamine 200mM; 0.02 % hydrocortisone; 0.1 % Insulin. Three hair follicles per well were then randomly distributed and cultured in 24 well plates (Costar, NY, USA) containing 500 ml of complete hair follicle culture medium per well.

Per experiment, a minimum of three wells (containing three hair follicles each) was assigned to each test group, and was supplemented with different concentrations of test substances pre-diluted in the respective dissolvent 100 x concentrated. An equal number of control follicles were cultured in complete hair follicle culture medium alone. Each experiment was repeated with hair follicles from at least three different age, pigmentation and sex matched donors.

Every second day, each well was photo-documented, the pigmentation degree of each hair follicle determined, medium replaced and fresh supplements added. After 7 days hair follicles were snap frozen and stored at -80 °C.

Steviol was tested at two different concentrations (0.625 and 1.25 µM). Macroscopic analysis of hair follicle pigmentation showed a slow loss of active pigmentation in the control group over the seven day culture period (in vitro graying) remaining pigmentation is scored minimal (+) in the table below. Sphingosyl phosphorylcholine at 1 µM was used as a positive control as it is known from previous work to reduce the speed of in vitro hair graying. Remaining pigmentation is scored (+++). As shown in the table below, a clear and significant improvement of hair follicle pigmentation was observed when steviol was used. This remaining pigmentation was superior when steviol was used at 0.625 µM (+++) when compared to steviol 1.25 µM (++).

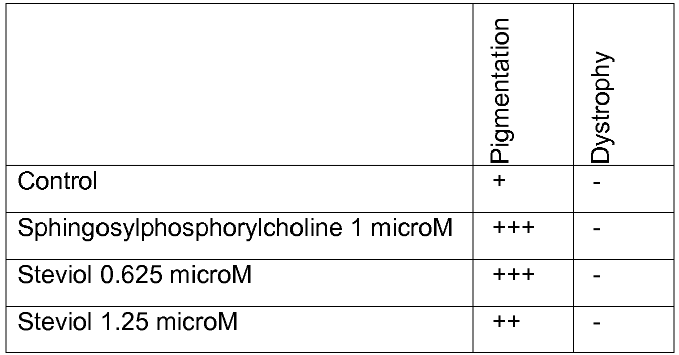

## Claims

1. Topical cosmetic, non-therapeutical use of a compound of formula (I) or a salt thereof, 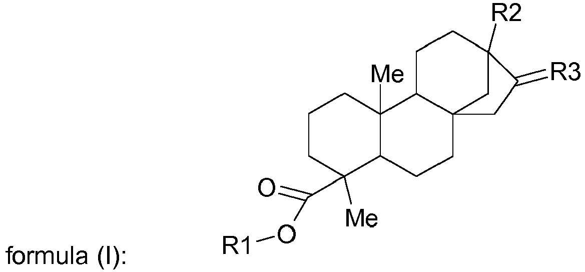 wherein
R1 is hydrogen (H)
R2 is hydroxyl (OH), and
R3 is CH₂
or
R1 is hydrogen (H)
R2 is methyl (CH₃), and
R3 is oxygen (O)
in a topical composition in an amount of 0.0001 to 5 wt.-%, based on the total weight of the composition, for the enhancement of the appearance of hair in an animal including human, wherein
the enhancement of the appearance of hair is selected from the group consisting of: restoring hair color and delaying the onset of greyness in hair, or delaying the onset or severity of age-associated hair loss and maintaining of the natural hair color.

2. Compound of formula (I) or a salt thereof, 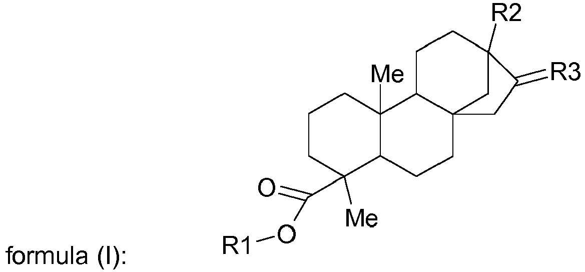 wherein
R1 is hydrogen (H)
R2 is hydroxyl (-OH), and
R3 is CH₂
or
R1 is hydrogen (H)
R2 is - methyl (CH₃), and
R3 is oxygen (O)
in a topical composition in an amount of 0.0001 to 5 wt.-%, based on the total weight of the composition, for the enhancement of the appearance of hair in an animal including human by topical application, wherein
the enhancement of the appearance of hair is restoring of hair growth after the onset of baldness has occurred.

3. The use according to claim 1 or the compound for use according to claim 2, wherein the compound of formula (I) is steviol or isosteviol with the stereochemistry as depicted in of formula (IV) and formula (V).

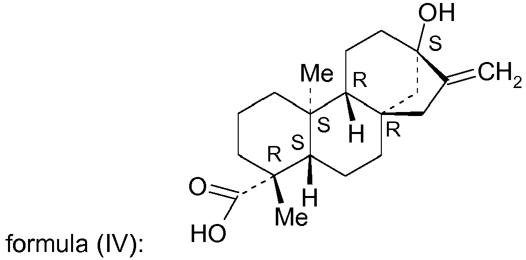

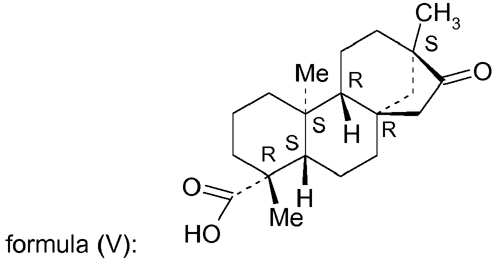

4. The use according to claim 1 or 3, wherein the enhancement of the appearance of hair is delaying the onset or severity of age-associated hair loss.

5. The use according to claim 1, 3 or 4 or the compound for use according to claim 2 or 3, wherein the compound of formula (I) as defined by any of the claims 1 to 3 is combined with the use of at least one additional active substance selected from the group consisting of antioxidants, light screening agents, colorants and biological actives selected from general activators of melanogenesis.

6. The use according to claims 1 and 3 to 5 or the compound for use according to claim 2, 3 and 5 wherein hair is a mammal's fur.

7. The use according to claims 1 and 3 to 7 or the compound for use according to claims 2, 3 and 5 to 7, wherein the amount of the compound of formula (I) is selected in the range of 0.001 to 0.5 weight- %, based on the total weight of the topical composition.

8. Cosmetic, non-therapeutic method for stimulating hair growth, and/or preventing the graying of hair or restoring or maintaining the natural hair color comprising the steps of applying to skin having hair, for a time, a topical composition comprising a compound as defined in claim 1 or 2 in an amount of 0.0001 to 5 wt.-%, based on the total weight of the composition and observing the result.

9. The method as in claim 8, wherein the amount of the compound of formula (I) is selected in the range of 0.001 to 0.5 weight- %, based on the total weight of the topical composition.

10. The method according to claim 8 or 9, wherein the topical composition is a hair care composition.

11. The method as in claim 10, wherein the hair care composition is a hair tonic, a conditioner, a shampoo, or a styling gel.

12. The method according to any one of claims 8 to 11, wherein the topical composition further comprises at least one additional active substance selected from the group consisting of antioxidants, light screening agents, colorants and biological actives.

## Patentansprüche

1. Topische kosmetische, nicht-therapeutische Verwendung einer Verbindung der Formel (I) oder eines Salzes davon, 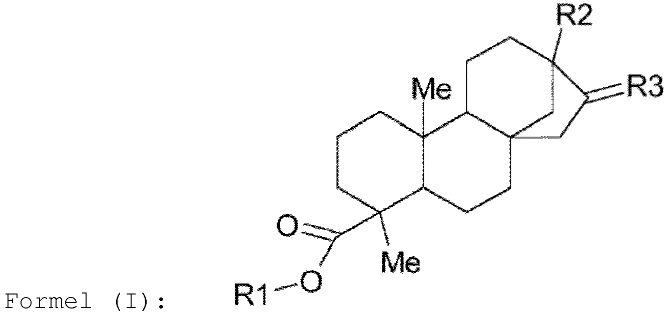 wobei
R1 für Wasserstoff (H) steht,
R2 für Hydroxyl (OH) steht, und
R3 für CH₂ steht
oder
R1 für Wasserstoff (H) steht,
R2 für Methyl (CH₃) steht, und
R3 für Sauerstoff (O) steht,
in einer topischen Zusammensetzung in einer Menge von 0,0001 bis 5 Gew.-%, bezogen auf das Gesamtgewicht der Zusammensetzung, zur Verbesserung des Aussehens von Haar in einem Tier, einschließlich Mensch, wobei
die Verbesserung des Aussehens von Haar ausgewählt ist aus der Gruppe, bestehend aus: Wiederherstellung der Haarfarbe und Verzögerung des Beginns des Ergrauens von Haar, oder Verzögerung des Beginns oder der Schwere von altersbedingtem Haarausfall, und Aufrechterhaltung der natürlichen Haarfarbe.

2. Verbindung der Formel (I) oder ein Salz davon, 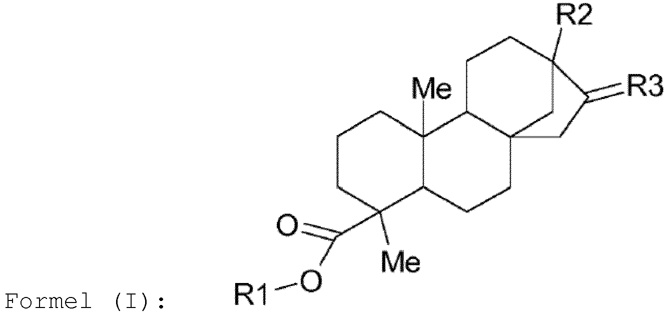 wobei
R1 für Wasserstoff (H) steht,
R2 für Hydroxyl (-OH) steht, und
R3 für CH₂ steht
oder
R1 für Wasserstoff (H) steht,
R2 für - Methyl (CH₃) steht, und
R3 für Sauerstoff (O) steht,
in einer topischen Zusammensetzung in einer Menge von 0,0001 bis 5 Gew.-%, bezogen auf das Gesamtgewicht der Zusammensetzung, zur Verbesserung des Aussehens von Haar in einem Tier, einschließlich Mensch, durch topische Applikation, wobei
die Verbesserung des Aussehens von Haar die Wiederherstellung des Haarwuchses ist, nachdem Haarlosigkeit begonnen hat.

3. Verwendung nach Anspruch 1, oder Verbindung zur Verwendung nach Anspruch 2, wobei die Verbindung der Formel (I) Steviol oder Isosteviol mit der bei Formel (IV) und Formel (V) gezeigten Stereochemie ist.

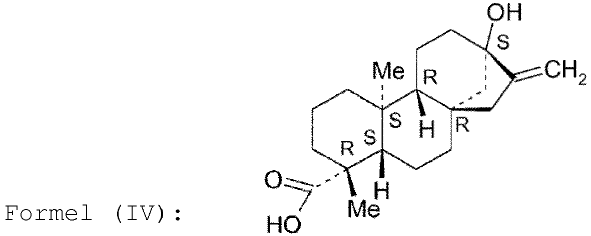

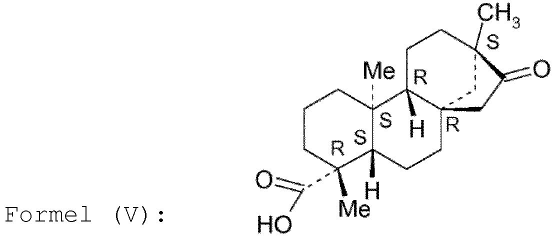

4. Verwendung nach Anspruch 1 oder 3, wobei die Verbesserung des Aussehens von Haar die Verzögerung des Beginns oder der Schwere von altersbedingtem Haarausfall ist.

5. Verwendung nach Anspruch 1, 3 oder 4, oder Verbindung zur Verwendung nach Anspruch 2 oder 3, wobei die Verbindung der Formel (I) nach einem der Ansprüche 1 bis 3 mit der Verwendung von mindestens einem zusätzlichen Wirkstoff kombiniert wird, ausgewählt aus der Gruppe, bestehend aus Antioxidantien, Lichtschutzmitteln, Farbstoffen und biologischen Wirkstoffen, ausgewählt aus allgemeinen Aktivatoren der Melanogenese.

6. Verwendung nach den Ansprüchen 1 und 3 bis 5, oder Verbindung zur Verwendung nach Anspruch 2, 3 und 5, wobei das Haar ein Pelz eines Säugetiers ist.

7. Verwendung nach den Ansprüchen 1 und 3 bis 7, oder Verbindung zur Verwendung nach den Ansprüchen 2, 3 und 5 bis 7, wobei die Menge der Verbindung der Formel (I) im Bereich von 0,001 bis 0,5 Gew.-%, bezogen auf das Gesamtgewicht der topischen Zusammensetzung, ausgewählt wird.

8. Kosmetisches, nicht-therapeutisches Verfahren zum Stimulieren des Haarwachstums, und/oder Verhindern des Ergrauens von Haar oder Wiederherstellen oder Aufrechterhalten der natürlichen Haarfarbe, umfassend die Schritte Auftragen einer topischen Zusammensetzung, die eine Verbindung nach Anspruch 1 oder 2 in einer Menge von 0,0001 bis 5 Gew.%, bezogen auf das Gesamtgewicht der Zusammensetzung, umfasst, auf Haar-aufweisende Haut für eine Zeitspanne, und Beobachten des Ergebnisses.

9. Verfahren nach Anspruch 8, wobei die Menge der Verbindung der Formel (I) im Bereich von 0,001 bis 0,5 Gew.-%, bezogen auf das Gesamtgewicht der topischen Zusammensetzung, gewählt wird.

10. Verfahren nach Anspruch 8 oder 9, wobei es sich bei der topischen Zusammensetzung um eine Haarpflege-Zusammensetzung handelt.

11. Verfahren nach Anspruch 10, wobei es sich bei der Haarpflege-Zusammensetzung um ein Haarwasser, einen Conditioner, ein Shampoo oder Stylinggel handelt.

12. Verfahren nach einem der Ansprüche 8 bis 11, wobei die topische Zusammensetzung zudem mindestens einen zusätzlichen Wirkstoff, ausgewählt aus der Gruppe, bestehend aus Antioxidantien, Lichtschutzmitteln, Farbstoffen und biologischen Wirkstoffen, umfasst.

## Revendications

1. Utilisation topique cosmétique non thérapeutique d'un composé de formule (I) ou d'un sel de celui-ci 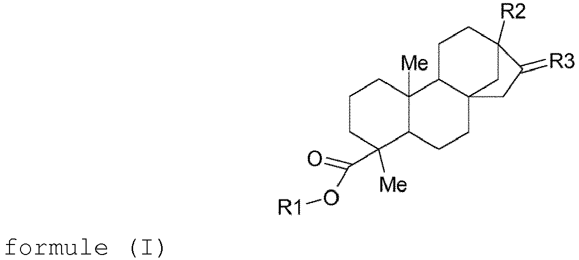 où
R1 est hydrogène (H),
R2 est hydroxyle (OH), et
R3 est CH₂,
ou
R1 est hydrogène (H),
R2 est méthyle (CH₃), et
R3 est oxygène (O),
dans une composition topique selon une quantité allant de 0,0001 à 5% en poids, sur la base du poids total de la composition, pour l'amélioration de l'aspect des poils ou des cheveux chez un animal, y compris l'homme, où l'amélioration de l'aspect des poils ou des cheveux est choisie dans le groupe constitué par la restauration de la couleur des poils ou des cheveux et le retardement du déclenchement du grisonnement des poils ou des cheveux, ou le retardement du déclenchement ou de la gravité de la perte des poils ou des cheveux associée à l'âge et le maintien de la couleur naturelle des poils ou des cheveux.

2. Composé de formule (I) ou un sel de celui-ci 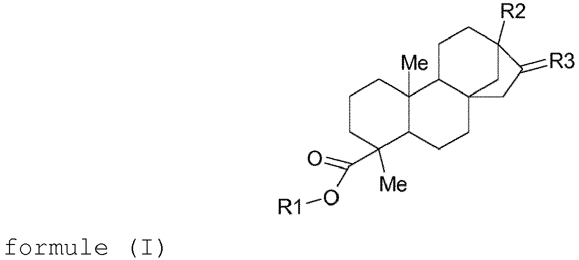 où
R1 est hydrogène (H),
R2 est hydroxyle (-OH), et
R3 est CH₂,
ou
R1 est hydrogène (H),
R2 est méthyle (CH₃), et
R3 est oxygène (O),
dans une composition topique selon une quantité allant de 0,0001 à 5% en poids, sur la base du poids total de la composition, pour l'amélioration de l'aspect des poils ou des cheveux chez un animal, y compris l'homme, par application topique, où
l'amélioration de l'aspect des poils ou des cheveux consiste à restaurer la croissance des poils ou des cheveux après que le déclenchement d'une calvitie s'est produit.

3. Utilisation selon la revendication 1 ou composé pour une utilisation selon la revendication 2, où le composé de formule (I) est le stéviol ou l'isostéviol ayant la stéréochimie telle qu'illustrée dans la formule (IV) et la formule (V).

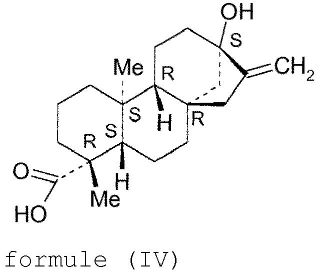

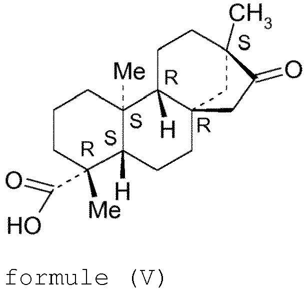

4. Utilisation selon la revendication 1 ou 3, dans laquelle l'amélioration de l'aspect des poils ou des cheveux consiste à retarder le déclenchement ou la gravité de la perte des poils ou des cheveux associée à l'âge.

5. Utilisation selon la revendication 1, 3 ou 4, ou composé pour une utilisation selon la revendication 2 ou 3, où le composé de formule (I) tel que défini selon l'une quelconque des revendications 1 à 3 est combiné avec l'utilisation d'au moins une substance active supplémentaire choisie dans le groupe constitué par les antioxydants, les agents photo-protecteurs, les colorants et les substances actives biologiques choisies parmi les activateurs généraux de la mélanogenèse.

6. Utilisation selon les revendications 1 et 3 à 5, ou composé pour une utilisation selon les revendications 2, 3 et 5, où les poils ou les cheveux sont la fourrure d'un mammifère.

7. Utilisation selon les revendications 1 et 3 à 7, ou composé pour une utilisation selon les revendications 2, 3 et 5 à 7, où la quantité de composé de formule (I) est choisie dans la plage allant de 0,001 à 0,5% en poids, sur la base du poids total de la composition topique.

8. Méthode cosmétique non thérapeutique destinée à stimuler la croissances des poils ou des cheveux, et/ou retarder le grisonnement des poils ou des cheveux, ou restaurer ou maintenir la couleur naturelle des poils ou des cheveux, comprenant les étapes consistant à appliquer à de la peau porteuse de poils ou de cheveux, pendant un certain temps, une composition topique comprenant un composé tel que défini selon la revendication 1 ou 2, selon une quantité allant de 0,0001 à 0,5% en poids, sur la base du poids total de la composition, et observer le résultat.

9. Méthode selon la revendication 8, dans laquelle la quantité de composé de formule (I) est choisie dans la plage allant de 0,001 à 0,5% en poids, sur la base du poids total de la composition topique.

10. Méthode selon la revendication 8 ou 9, dans laquelle la composition topique est une composition de soin des cheveux.

11. Méthode selon la revendication 10, dans laquelle la composition de soin des cheveux est une lotion tonique capillaire, un après-shampooing, un shampooing, ou un gel de coiffage.

12. Méthode selon l'une quelconque des revendications 8 à 11, dans laquelle la composition topique comprend en outre au moins une substance active supplémentaire choisie dans le groupe constitué par les antioxydants, les agents photo-protecteurs, les colorants et les substances actives biologiques.
